# EUROPÄISCHE PATENTSCHRIFT

(11) **EP 3 534 377 B1**
(45) Veröffentlichungstag und Bekanntmachung des Hinweises auf die Patenterteilung: **17.11.2021**
(21) Anmeldenummer: 18159259.3
(22) Anmeldetag: 28.02.2018
(51) Int. Cl.: G21K 1/02, G21K 1/06

(54) **VERFAHREN ZUR HERSTELLUNG EINES MIKROSTRUKTURBAUTEILS**
METHOD FOR PRODUCING A MICROSTRUCTURE COMPONENT
PROCÉDÉ DE FABRICATION D'UN COMPOSANT MICROSTRUCTURÉ

(43) Veröffentlichungstag der Anmeldung: 04.09.2019
(73) Patentinhaber: Siemens Healthcare GmbH, 91052 Erlangen (DE)
(72) Erfinder: DEUTINGER, Andrea, 85659 Forstern (DE); ZAPF, Jörg, 81927 München (DE)

(56) Entgegenhaltungen:
- A. V. CHERNIENKO ET AL: "Zigzag structures obtained by anisotropic etching of macroporous silicon", TECHNICAL PHYSICS LETTERS., Bd. 39, Nr. 11, 1. November 2013 (2013-11-01), Seiten 990-993, XP55502266, US ISSN: 1063-7850, DOI: 10.1134/S1063785013110175
- ASTROVA E V ET AL: "Structure and composition of silicon microarrays subjected to cyclic insertion and extraction of lithium", TECHNICAL PHYSICS, PLEIADES PUBLISHING / AIP, MELVILLE, NY, US, Bd. 60, Nr. 4, 29. April 2015 (2015-04-29) , Seiten 531-540, XP035492269, ISSN: 1063-7842, DOI: 10.1134/S1063784215040040 [gefunden am 2015-04-29]

## Beschreibung

Die vorliegende Erfindung betrifft ein Verfahren zur Herstellung eines Mikrostrukturbauteils, insbesondere eines Röntgenphasenkontrastgitters.

Im Bereich der Röntgenbildgebung insbesondere im medizinischen Bereich, wird teilweise der sogenannte Talbot-Effekt genutzt. Hierdurch können präzisere Bildinformationen generiert werden, indem der Kontrast der erzeugten Bilder durch Einbeziehung einer von einem Untersuchungsobjekt verursachten Phasenverschiebung der Röntgenstrahlen verbessert wird. Dabei kommt regelmäßig ein sogenanntes (Röntgen-)Phasenkontrastgitter zum Einsatz, das in den Strahlengang eingebracht wird. Ein solches Gitter ist dabei üblicherweise durch in Röntgen-Strahlungsrichtung ausgerichtete Lamellen aus einem röntgenstrahlenabsorbierenden Material gebildet. Üblicherweise liegen die Wandstärke und die Abstände dieser Lamellen zueinander dabei im ein- bis gering zweistelligen Mikrometerbereich. Auch die Dicke (oder: Höhe) des gesamten Gitters liegt im durchstrahlten Bereich bei meist maximal einem Millimeter. Somit handelt es sich bei einem solchen Phasenkontrastgitter um ein Mikrostrukturbauteil.

Aufgrund der geringen Abmessungen werden diese Phasenkontrastgitter meist durch Ätzverfahren in Siliziumwafern und nachträglichem Füllen der geätzten Vertiefungen mit Röntgenstrahlen absorbierendem Material zur Ausbildung der Lamellen hergestellt. Dies ist beispielsweise aus DE 10 2015 201 741 A1 bekannt. Problematisch ist dabei häufig, dass die Phasenkontrastgitter idealerweise an die lokale Strahlungsrichtung der von einer meist punktförmigen Strahlungsquelle ausgehenden Radialstrahlen angepasst sein sollten. D. h. die Lamellen sollten idealerweise in einem Winkel zueinander angestellt sein.

Ein Ätzverfahren zur Ausbildung von Membranen oder Elektroden aus Silizium ist beispielsweise aus A. V. Chernienko et al.: "Zigzag Structures Obtained by Anisotropic Etching of Macroporous Silicon", Technical Physics Letters, 2013, Vol. 39, No. 11, pp. 990-993, ISSN 1063-7850, DOI: 10.1134/S1063785013110175 sowie aus E. V. Astrova et al.: "Structure and Composition of Silicon Microarrays Subjected to Cyclic Insertion and Extraction of Lithium", Technical Physics, 2015, Vol. 60, No. 4, pp. 531-540, ISSN 1063-7842, DOI: 10.1134/S1063784215040040 bekannt.

Der Erfindung liegt die Aufgabe zugrunde, ein verbessertes Mikrostrukturbauteil zu ermöglichen.

Diese Aufgabe wird erfindungsgemäß gelöst durch ein Verfahren zur Herstellung eines solchen Mikrostrukturbauteils mit den Merkmalen des Anspruchs 1. Vorteilhafte Ausführungsformen und Weiterbildungen der Erfindung sind in den Unteransprüchen und der nachfolgenden Beschreibung dargelegt.

Das erfindungsgemäße Verfahren dient zur Herstellung eines Mikrostrukturbauteils. Verfahrensgemäß wird dabei in eine erste Oberfläche eines scheibenartigen Siliziumsubstrats, insbesondere in einen Wafer oder einen Teil eines Wafers, eine Vielzahl von punktartigen und insbesondere voneinander separaten Impfstrukturen in einem Raster, das entlang einer ersten und einer zweiten - zur ersten senkrecht stehenden - Substratrichtung vorgegeben ist, eingebracht. Darauf folgend werden die Impfstrukturen (vorzugsweise nur) in Tiefenrichtung (oder auch: Dickenrichtung) des Siliziumsubstrats in einem ersten Ätzschritt zu Bohrlöchern verlängert - d. h. vertieft. Anschließend wird eine der ersten Oberfläche des Siliziumsubstrats gegenüberliegende zweite Oberfläche zur rückseitigen Öffnung der Bohrlöcher in einem zweiten Ätzschritt zumindest teilweise entfernt. In einem dritten Ätzschritt wird dann ein anisotrop wirksames Ätzmedium - insbesondere eine Ätzlösung - alternierend von beiden Oberflächen des Siliziumsubstrats her durch die Bohrlöcher gespült. Das Ätzmedium wird dabei so lange alternierend durch die Bohrlöcher gespült, bis sich die in der ersten Substratrichtung nebeneinander angeordneten Bohrlöcher zu einem in der ersten Substratrichtung verlaufenden Spalt verbinden.

Vorzugsweise wird durch das vorstehend beschriebene Verfahren als Mikrostrukturbauteil insbesondere ein (Röntgen-)Phasenkontrastgitter hergestellt.

Weiter vorzugsweise werden als erste und zweite Substratrichtung Richtungen herangezogen, entlang derer als "(111)-Kristallebenen" bezeichnete Kristallebenen des Siliziumsubstrats durch eine parallel zur ersten Oberfläche ausgerichtete "(100)-Kristallebene" des Siliziumsubstrats hindurch treten.

Dadurch, dass in dem ersten Ätzschritt die Impfstrukturen in Dickenrichtung - vorzugsweise mit keiner, lediglich geringfügiger oder vernachlässigbarer Aufweitung - zu den Bohrlöchern vertieft werden, können diese vorteilhafterweise mit einem vergleichsweise hohen Durchmesser- zu Tiefenverhältnis (auch: "Aspektverhältnis") ausgeformt werden. Da in dem dritten Ätzschritt mehrere nebeneinander angeordnete Bohrlöcher zu dem Spalt verbunden werden, braucht lediglich das Substratmaterial zwischen zwei in der ersten Substratrichtung benachbarten Bohrlöchern abgetragen werden, wodurch vorteilhafterweise ermöglicht wird, auch das Aspektverhältnis (d. h. das Breitenzu Höhenverhältnis) der aus den Bohrlöchern gebildeten Spalte möglichst hoch zu halten - vorzugsweise also nur eine vergleichsweise geringfügige "Verbreiterung" in der zweiten Substratrichtung zuzulassen. Vorzugsweise sind hierzu über die bestimmungsgemäße Längserstreckung eines jeden Spalts mehrere Impfstrukturen und somit auch mehrere Bohrlöcher verteilt. Ein hohes Aspektverhältnis ist dabei regelmäßig für besonders präzise Abbildungseigenschaften beim Einsatz des Mikrostrukturbauteils in einem Röntgengerät als (Röntgen-) Phasenkontrastgitter vorteilhaft.

Des Weiteren werden die Impfstrukturen in der zweiten Substratrichtung mit einem ersten Abstand zueinander angeordnet. In der ersten Substratrichtung werden sie jeweils zu Gruppen zusammengefasst. Diese Gruppen umfassen jeweils eine Unteranzahl der Impfstrukturen. Außerdem werden die Impfstrukturen innerhalb dieser Gruppen mit einem um einen Faktor von 0,2-0,6, vorzugsweise von 0,3-0,5 gegenüber dem ersten Abstand verringerten zweiten Abstand zueinander angeordnet. D. h. die Impfstrukturen weisen innerhalb der Gruppen und somit entlang der ersten Substratrichtung einen Abstand auf, der um wenigstens 40 Prozent gegenüber dem ersten Abstand kleiner ist. Dadurch wird auf einfache Weise ermöglicht, dass - wie vorstehend beschrieben - die Spalte in dem dritten Ätzschritt sich in der ersten Substratrichtung durch Verbindung der einzelnen Bohrlöcher in vergleichsweise kurzer Zeit ausbilden und in der zweiten Substratrichtung jeweils durch einen Steg (im Folgenden als "Längssteg" bezeichnet) voneinander getrennt sind (sowie vorteilhafterweise im Vergleich zur Längserstreckung der Spalte nur eine geringfügige Verbreiterung erfahren).

Zusätzlich werden die vorstehend beschriebenen Gruppen in der ersten Substratrichtung mit einem gegenüber dem zweiten Abstand vergrößerten dritten Abstand aufeinanderfolgend angeordnet. D. h. die Gruppen werden in der ersten Raumrichtung räumlich voneinander (weiter als die Impfstrukturen innerhalb der Gruppen) getrennt. Der dritte Abstand wird dabei derart gewählt, dass in dem dritten Ätzschritt die randseitigen Impfstrukturen einer jeden Gruppe nicht mit den Impfstrukturen der in der ersten Substratrichtung nächstliegenden Gruppe zusammenwachsen. Insbesondere wird dadurch vorteilhafterweise ermöglicht, dass sich in dem dritten Ätzschritt in der ersten Substratrichtung gesehen mehrere aufeinanderfolgende Spalte ausbilden, die jeweils durch einen Quersteg, der in Richtung der zweiten Substratrichtung ausgerichtet ist, voneinander getrennt sind. Dadurch wird die mechanische Stabilität (insbesondere eine Biegesteifigkeit) des mit den Spalten durchsetzten Siliziumsubstrats in der zweiten Substratrichtung gesehen vorteilhaft erhöht.

In einer zweckmäßigen Verfahrensvariante werden die vorstehend beschriebenen Gruppen, die in der zweiten Substratrichtung gesehen aufeinander folgen, bezüglich ihrer Längserstreckung entlang der ersten Substratrichtung insbesondere alternierend zueinander versetzt angeordnet. D. h. eine jede Gruppe und somit ein jeder daraus gebildeter Spalt ist gegenüber der in der zweiten Substratrichtung benachbarten Gruppe bzw. dem benachbarten Spalt in der ersten Substratrichtung verschoben angeordnet. In diesem Fall - insbesondere im Fall der vorstehend beschriebenen Querstege - "mäandern" die in dem dritten Ätzschritt ausgebildeten Querstege in der zweiten Substratrichtung gesehen um die in der ersten Substratrichtung verlaufenden Spalte (d. h. die Querstege liegen in der zweiten Substratrichtung gesehen nicht, zumindest nicht alle in einer Flucht). Dadurch ergibt sich vorteilhaft, dass die mechanische Stabilität des gebildeten Mikrostrukturbauteils gegen eine Biegung um eine in der ersten Substratrichtung verlaufende Biegeachse zwar insbesondere gegenüber Spalte, die in der ersten Substratrichtung durchgehend verlaufen, erhöht ist, aber dass dennoch eine für eine Biegung um diese Biegeachse hinreichende Flexibilität ermöglicht wird. Letzteres ist vorteilhaft, um - insbesondere im Fall des Phasenkontrastgitters - eine Anpassung der vorzugsweise in den Spalten ausgebildeten Gitter-Lamellen an die lokale Strahlungsrichtung zu ermöglichen, insbesondere das Phasenkontrastgitter auf einfache Weise um diese Biegeachse (vorzugsweise entlang einer kreiszylindrischen Fläche) biegen zu können.

In einer in fertigungstechnischer Hinsicht zweckmäßigen Weiterbildung werden die vorstehend beschriebenen Gruppen um etwa (vorzugsweise genau) die Hälfte ihrer Längserstreckung entlang der ersten Substratrichtung zueinander versetzt angeordnet. Mithin werden die in den in der ersten Substratrichtung verlaufenden "Spalt-Zeilen" liegenden Gruppen bei jedem Zeilensprung (d. h. in jeder in der zweiten Substratrichtung benachbarten Spalt-Zeile) um etwa die Hälfte in Längsrichtung verschoben. Dadurch wird vorzugsweise eine symmetrische Abfolge der Spalte in der zweiten Substratrichtung ermöglicht, die wiederum vorteilhafterweise zu einer möglichst gleichmä-βigen Deformation bei einer Biegung des Siliziumsubstrats um die erste Substratrichtung führt.

In einer weiteren zweckmäßigen Verfahrensvariante wird für die Impfstrukturen eine quadratische Grundfläche mit einem Durchmesser bzw. eine Kantenlänge kleiner oder gleich 2,5 Mikrometer, insbesondere kleiner oder gleich 2 Mikrometer herangezogen.

In einer bevorzugten Verfahrensvariante werden für den ersten Abstand Werte zwischen 2-20 Mikrometer, insbesondere bis etwa 12 Mikrometer herangezogen. Vorzugsweise entspricht dieser erste Abstand dabei einer im bestimmungsgemäßen Einsatzzustand des Mikrostrukturbauteils als Phasenkontrastgitter geforderten Gitterkonstanten.

In einer weiteren bevorzugten Verfahrensvariante werden für die Längserstreckung der Gruppen Werte zwischen 40 und 1300 Mikrometer, insbesondere zwischen 40 und 500 Mikrometer, besonders bevorzugt zwischen 50 und 300 Mikrometer herangezogen. Insbesondere bei Längserstreckungen unter 1000 Mikrometern wird vorteilhafterweise eine hinreichend hohe mechanische Stabilität des Mikrostrukturbauteils ermöglicht.

In einer weiteren zweckmäßigen Verfahrensvariante werden die Bohrlöcher bis zu einer Tiefe von etwa 400 bis 500 Mikrometern, bspw. 450 oder 470 Mikrometern, ausgeformt ("vorangetrieben"). Dadurch ergibt sich insbesondere in Verbindung mit den vorstehend beschriebenen Abmessungen der Impfstrukturen ein besonders hohes Aspektverhältnis der Bohrlöcher und somit auch der daraus gebildeten Spalte. Vorzugsweise wird in dem ersten Ätzschritt dabei das sogenannte PAECE-Verfahren ("photo assisted electro-chemical etching") eingesetzt. Vorzugsweise wird zur Unterstützung dieses Ätzverfahrens eine entsprechende Dotierung für das Siliziumsubstrat gewählt. Die Bohrlöcher weisen nach dem Ätzen insbesondere eine kreiszylindrische Struktur, d. h. eine kreisförmige Grundfläche, auf.

In einer bevorzugten Verfahrensvariante wird als das in dem dritten Ätzschritt eingesetztes anisotrop wirksames Ätzmedium eine Lösung gewählt, die Kaliumhydroxid (KOH), Wasserstoffperoxid und Isopropanol enthält. Dadurch wird insbesondere erreicht, dass die Bohrlöcher von ihrer kreisförmigen Grundfläche zu einer quadratischen Grundfläche übergehen und somit zu quadratischen "Zylinderlöchern" aufwachsen. Insbesondere bei exakter Ausrichtung der ersten und zweiten Substratrichtung an dem Verlauf der (111)-Kristallebenen des Siliziumsubstrats verlaufen die Kanten der aufgeweiteten Bohrlöcher ebenfalls exakt entlang der ersten und zweiten Substratrichtung, so dass die gebildeten Spalte geradlinige Kanten entlang dieser beiden Substratrichtungen aufweisen.

In einer weiteren zweckmäßigen Verfahrensvariante werden die Impfstrukturen insbesondere durch Ätzen mittels einer auf die erste Oberfläche aufgebrachten Maskier-Schicht, beispielsweise einer fotolithographischen Schicht und/oder einer keramischen Schutzschicht, in die erste Oberfläche eingebracht. Die Impfstrukturen werden dabei insbesondere nach einer lokalen Öffnung der Maskier-Schicht in Form von kreisförmigen oder auf die Zielkontur der Impfstrukturen abgestimmten quadratischen "Löchern" mittels einer Lauge, insbesondere einer Kaliumhydroxid enthaltenden Lösung, in die erste Oberfläche des Siliziumsubstrats übertragen. Insbesondere werden die Impfstrukturen dabei in dem Siliziumsubstrat in Tiefenrichtung gesehen mit einer pyramidalen Struktur mit quadratischer Grundfläche ausgebildet. Die Maskier-Schicht wird vorzugsweise nach dem Einbringen der Impfstrukturen wieder entfernt.

Insbesondere für den Fall, dass es sich bei dem Mikrostrukturbauteil um das Phasenkontrastgitter handelt, wird dieses nach dem dritten Ätzschritt - d. h. nach dem Aufweiten der Bohrlöcher zu dem jeweiligen Spalt - um eine entlang der ersten Substratrichtung liegende Achse (d. h. um die vorstehend beschriebene Biegeachse) gebogen. Der Biegeradius wird dabei vorzugsweise derart gewählt, dass die Tiefenrichtung eines jeden Spalts (oder die Höhenrichtung der zwischen diesen liegenden Längsstege) parallel zu einer im bestimmungsgemäßen Einsatzzustand des Phasenkontrastgitters auftretenden lokalen Strahlungsrichtung eines das Phasenkontrastgitter durchstrahlenden Röntgenstrahls ausgerichtet ist. Der Biegeradius kann dabei weniger als einen Zentimeter betragen.

Vorzugsweise werden die Spalte insbesondere nach dem vorstehend beschriebenen Biegen mit einem Röntgenstrahlen absorbierenden Material, insbesondere einer Metalllegierung gefüllt. Beispielsweise kommt dabei Zinn oder eine Zinnlegierung, Tantal, Wolfram, Gold oder dergleichen zum Einsatz. Beispielsweise erfolgt die Füllung durch einen Gießprozess, durch galvanische oder stromlose Abscheidungsprozesse.

In einer zweckmäßigen Verfahrensvariante werden in einem zusätzlichen (vierten) Ätzschritt in der Rückseite (d. h. der zweiten Oberfläche) des Siliziumsubstrats ein insbesondere in der zweiten Substratrichtung gesehen zentraler Bereich des Siliziumsubstrats (vorzugsweise über die komplette, sich in der ersten Substratrichtung erstreckende Fläche) entfernt und Seitenbereiche der zweiten Oberfläche, die in der zweiten Substratrichtung gesehen endständig zu dem Siliziumsubstrat angeordnet sind, zumindest teilweise belassen werden. Mit anderen Worten handelt es sich bei diesen Seitenbereichen um Randbereiche des Siliziumsubstrats, die in diesem Ätzschritt nicht oder gegenüber dem zentralen Bereich nur geringfügig abgetragen werden. Vorzugsweise werden die Seitenbereiche dabei entsprechend maskiert, so dass das hierbei eingesetzte Ätzmedium diese Seitenbereiche nicht "angreifen" kann. Durch den zentralen Abtrag wird die Biegesteifigkeit des Siliziumsubstrats weiter verringert. Die Randbereiche ergeben au-βerdem eine "Aufdickung" des Siliziumsubstrats, die erkanntermaßen eine erhöhte mechanische Stabilität aufweist und als Handlingstruktur dienen kann. Insbesondere für den Einsatz des Mikrostrukturbauteils als Phasenkontrastgitter wird in dem zentralen Bereich (der vorzugsweise einem durchstrahlten Bereich entspricht) auch eine Absorption der Röntgenstrahlen im Silizium verringert. Vorzugsweise erfolgt dieser vierte Ätzschritt nach einer Füllung der Spalte mit dem Röntgenstrahlen absorbierenden Material, so dass dieses Material in dem zentralen Bereich zumindest teilweise in Form von freistehenden Lamellen aus dem verbleibenden Siliziumsubstrat hervorsteht.

Das so erhaltene Mikrostrukturbauteil, insbesondere das durch dieses gebildete Phasenkontrastgitter, ist also gemäß dem vorstehend beschriebenen Verfahren hergestellt. Vorzugsweise ist das Mikrostrukturbauteil im Fall des Phasenkontrastgitters auch gebogen. Somit weist das Mikrostrukturbauteil die gleichen, sich aus dem vorstehend beschriebenen Verfahren ergebenden Merkmale und Vorteile auf.

In hinsichtlich der mechanischen Stabilität vorteilhafter Weise weist das Mikrostrukturbauteil eine Vielzahl der in der ersten Substratrichtung längserstreckten (in Dickenrichtung durchgängigen) Spalte auf. In der zweiten Substratrichtung gesehen sind diese Spalte ggf. von Spalt-Zeile zu Spalt-Zeile hinsichtlich ihrer Längserstreckung zueinander versetzt angeordnet.

Vorzugsweise sind diese Spalte mit dem Röntgenstrahlen absorbierenden Material gefüllt.

Ein Röntgengerät, welches das durch das vorstehend beschriebene Mikrostrukturbauteil gebildete Phasenkontrastgitter aufweist, teilt mithin auch die vorstehend beschriebenen Merkmale und Vorteile. Insbesondere sind im bestimmungsgemäßen Einsatzzustand im Röntgengerät die vorstehend beschriebenen Spalte des Mikrostrukturbauteils mit dem Röntgenstrahlen absorbierenden Material zur Bildung der Gitter-Lamellen gefüllt.

Nachfolgend werden Ausführungsbeispiele der Erfindung und weitere, nicht zur beanspruchten Erfindung gehörige Beispiele anhand einer Zeichnung näher erläutert. Darin zeigen:
- FIG 1: in einer schematischen Seitenansicht ein Röntgengerät mit einem Phasenkontrastgitter,
- FIG 2: in einem schematischen Ablaufdiagramm ein Verfahren zur Herstellung des Phasenkontrastgitters,
- FIG 3, 4: in einer schematischen Draufsicht jeweils Raster von Impfstrukturen zur Erzeugung des Phasenkontrastgitters aus einem Siliziumsubstrat,
- FIG 5: in einem schematischen Querschnitt durch das Siliziumsubstrat einen Ätzschritt,
- FIG 6: in Ansicht gemäß FIG 3 aus dem Raster gebildete Spalte in dem Siliziumsubstrat, und
- FIG 7: in Ansicht gemäß FIG 5 ein optionales Phasenkontrastgitter.

Einander entsprechende Teile sind in allen Figuren stets mit gleichen Bezugszeichen versehen

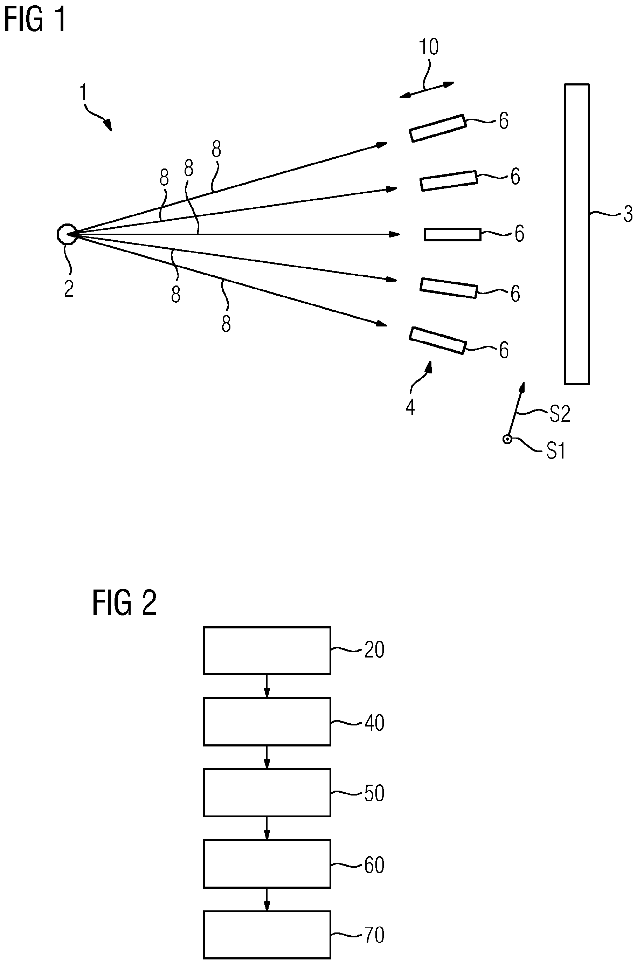

In FIG 1 ist schematisch angedeutet ein Röntgengerät 1 dargestellt. Das Röntgengerät 1 weist eine Röntgen-Strahlenquelle 2 und einen Röntgen-Strahlendetektor 3 auf. Im Strahlengang zwischen der Röntgen-Strahlenquelle 2 und dem Röntgen-Strahlendetektor 3 ist ein (Röntgen-) Phasenkontrastgitter 4 angeordnet. Das Phasenkontrastgitter 4 weist dabei eine Anzahl von Lamellen 6 auf, die parallel zu dem jeweiligen lokalen Röntgen-Teilstrahl 8 ausgerichtet sind. Das Phasenkontrastgitter 4 weist dabei in Dickenrichtung 10 (oder auch: in Strahlungsrichtung) gesehen Abmessungen von maximal einem Millimeter auf. Somit handelt es sich bei dem Phasenkontrastgitter 4 um ein Bauteil mit Strukturen im Mikrometerbereich ("Mikrostrukturbauteil"). Um bei solchen geringen Abmessungen dennoch die Lamellen 6 des Phasenkontrastgitters 4 präzise abformen zu können, wird ein im Folgenden anhand von FIG 2 näher beschriebenes Herstellungsverfahren durchgeführt.

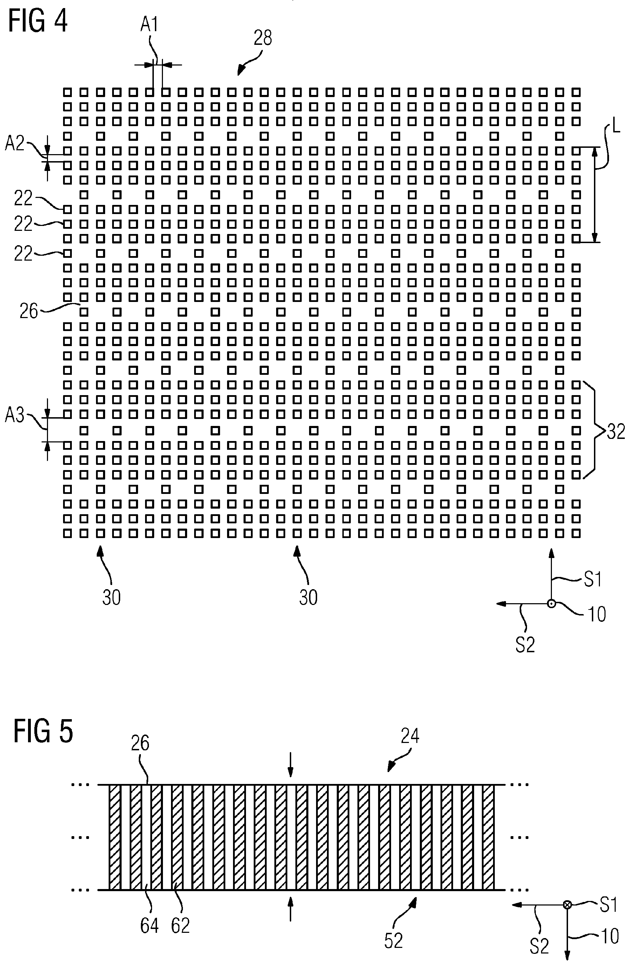

In einem ersten Verfahrensschritt 20 werden dabei Impfstrukturen 22 (vergleiche FIG 4) in eine erste Oberfläche 24 (s. FIG 5) eines scheibenartigen Siliziumsubstrats 26 eingebracht. Bei dem Siliziumsubstrat 26 handelt es sich konkret um einen Silizium-Wafer. Die Impfstrukturen 22 sind dabei in einem Raster 28 über die erste Oberfläche 24 des Siliziumsubstrats 26 verteilt. Das Raster 28 ist dabei (konkret zeilenartig) in einer ersten Substratrichtung S1 und einer zweiten Substratrichtung S2 vorgegeben. D. h. die Impfstrukturen 22 werden in sogenannten "Spalt-Zeilen" 30, die in der ersten Substratrichtung S1 verlaufen und sich in der zweiten Substratrichtung S2 wiederholen, angeordnet. In Zeilenrichtung - d. h. in der ersten Substratrichtung S1 - sind jeweils mehrere Impfstrukturen 22 (im vorliegenden Ausführungsbeispiel konkret sieben Impfstrukturen 22) zu jeweils einer Gruppe 32 zusammengefasst. Die jeweiligen Spalt-Zeilen 30, konkret die in der zweiten Substratrichtung S2 nebeneinander angeordneten Impfstrukturen 22 werden dabei mit einem ersten Abstand A1 zueinander angeordnet. Der erste Abstand A1 beträgt im vorliegenden Ausführungsbeispiel 12 Mikrometer und entspricht dabei konkret einer Gitterkonstanten des Phasenkontrastgitters 4. Innerhalb der Gruppen 32 sind die Impfstrukturen 22 mit einem zweiten Abstand A2 zueinander angeordnet, der um den Faktor 0,5 kleiner ist als der erste Abstand A1.

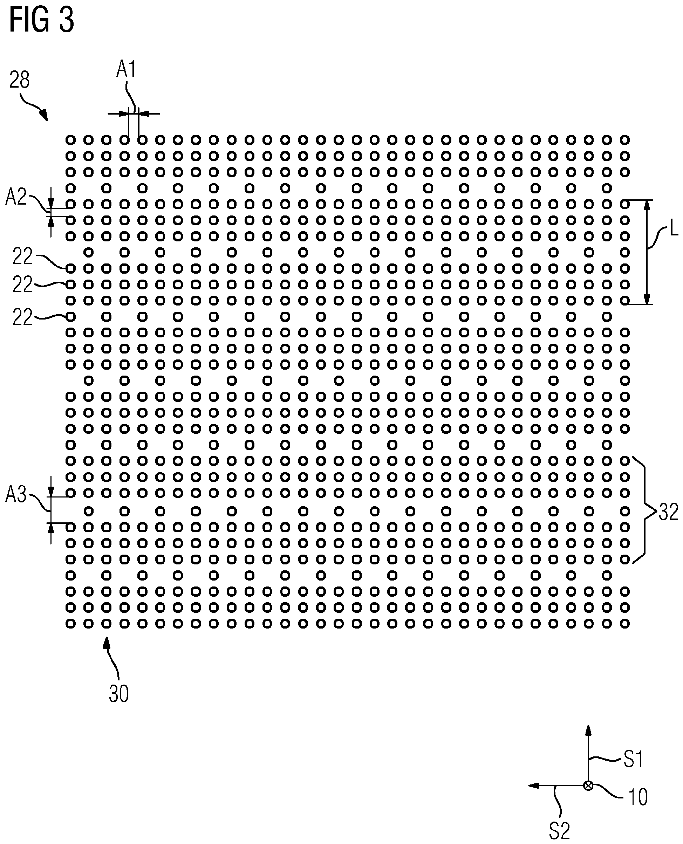

Die Impfstrukturen 22 werden hierzu zunächst als Löcher in einer Maskier-Schicht (nicht näher dargestellt) abgebildet, bspw. wie in FIG 3 gezeigt kreisförmig oder bereits mit der in FIG 4 dargestellten quadratischen Grundfläche. Anschlie-βend werden die Impfstrukturen durch Ätzen auf die erste Oberfläche 24 des Siliziumsubstrats 26 übertragen. Dort sind sie stets mit quadratischer Grundfläche (auch im Fall der kreisförmigen Maskierung) abgeformt. Die Impfstrukturen 22 werden dabei mit einer Kantenlänge von kleiner oder gleich 2 Mikrometern ausgebildet. Eine Längserstreckung L der Gruppen 32 beträgt somit etwa 50 Mikrometer.

Die Gruppen 32 sind außerdem mit einem dritten Abstand A3 zueinander beabstandet angeordnet. Der dritte Abstand A3 entspricht dabei etwa einer Auslassung einer der Impfstrukturen 22 in der jeweiligen Spalt-Zeile 30.

In einem zweiten Verfahrensschritt 40 werden in einem Ätzschritt die Impfstrukturen 22 in Dickenrichtung 10 zu runden Bohrlöchern erweitert, deren Seitenwände senkrecht zu der ersten Oberfläche 24 stehen. Dazu wird das sogenannte PAECE-Verfahren eingesetzt. Konkret werden die Impfstrukturen 22 zu Bohrlöchern mit einer Tiefe von 470 Mikrometern erweitert.

In einem dritten Verfahrensschritt 50 wird in einem weiteren Ätzschritt die der ersten Oberfläche 24 gegenüberliegende zweite Oberfläche 52 derart entfernt, dass die Bohrlöcher rückseitig (d. h. zu der zweiten Oberfläche 52 hin) geöffnet sind. Dazu kommen Ätzverfahren wie zum Beispiel nasschemisches Ätzen mit Kaliumhydroxid oder plasmainduziertes Trockenätzen zum Einsatz.

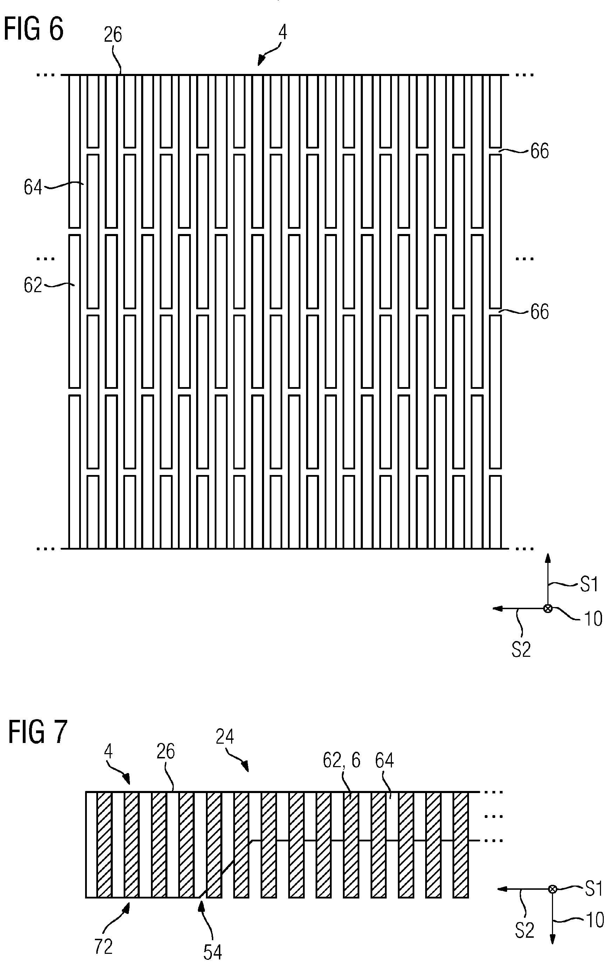

In einem vierten Verfahrensschritt 60 werden in einem erneuten Ätzschritt die Bohrlöcher alternierend von der ersten Oberfläche 24 und der zweiten Oberfläche 52 her mit einem anisotrop wirksamen Ätzmedium durchspült (vgl. entgegengesetzte Pfeile in FIG 5). Das Ätzmedium enthält dabei Kaliumhydroxid, Wasserstoffperoxid und Isopropanol. Die erste und die zweite Substratrichtung S1 bzw. S2 sind parallel zu Richtungen gewählt, in denen (111)-Kristallebenen des Siliziums durch die (parallel zur ersten Oberfläche 24 liegenden) (100)-Kristallebene hindurch treten. Dadurch werden die einzelnen Bohrlöcher in der ersten und zweiten Substratrichtung S1 und S2 aufgeweitet, so dass sie einen quadratischen Querschnitt aufweisen, und vereinigen sich bei fortschreitender Aufweitung mit den benachbarten Bohrlöchern innerhalb der jeweiligen Gruppen 32 zu jeweils einem Spalt 62 (s. FIG 5, 6). Da der erste Abstand A1 größer ist als der zweite Abstand A2 verbleiben nach der Spaltbildung zwischen den einzelnen Spalt-Zeilen 30 Längsstege 64 aus Silizium. Aufgrund des dritten Abstands A3 zwischen den einzelnen Gruppen 32 verbleibt auch in der ersten Substratrichtung S1 gesehen zwischen den durch die Gruppen 32 gebildeten Spalten 62 jeweils ein Quersteg 66.

Das Raster 28 ist dabei derart gewählt, dass die Gruppen 32 zweier direkt benachbarter Spalt-Zeilen 30 um die Hälfte der Längserstreckung L in der ersten Substratrichtung S1 versetzt sind. Dadurch fluchten die Querstege 66 zweier direkt benachbarter Spalt-Zeilen 30 nicht miteinander. Vielmehr fluchten die Querstege 66 immer der "übernächsten" Spalt-Zeilen 30 miteinander. Dadurch ergibt sich ein guter Kompromiss von mechanischer Stabilität gegen eine Biegung um eine in der ersten Substratrichtung S1 gerichtete Biegeachse und einer hinreichenden Flexibilität für eine solche Biegung.

In einem weiteren Verfahrensschritt 70 wird das Siliziumsubstrat 26 um eine in der ersten Substratrichtung S1 liegende Biegeachse gebogen. Anschließend werden die Spalte 62 mit einem metallischen Werkstoff, konkret eine Zinnlegierung gefüllt. Die gefüllten Spalte 62 ergeben dabei die in FIG 1 dargestellten Lamellen 6.

In einem in FIG 7 dargestellten Phasenkontrastgitter werden in einem weiteren Ätzschritt (in einem zusätzlichen, optionalen Verfahrensschritt) zentrale Bereiche der Rückseite (d. h. auf der Seite der zweiten Oberfläche 52) des Siliziumsubstrats 26 in Dickenrichtung 10 teilweise entfernt. Nur in der zweiten Substratrichtung S2 verbleiben endständige Randbereiche 72 (auch: Seitenbereiche), so dass in diesen Randbereichen 72 eine massivere und damit mechanisch stabilere Handlingstruktur ("Griffstruktur") vorhanden ist. Konkret werden die Randbereiche 72 vor dem Ätzen entsprechend maskiert. Das Ätzen erfolgt mittels Kaliumhydroxid. Die durch die Zinnlegierung in den gefüllten Spalten 62 gebildeten Lamellen 6 stehen hiernach zumindest teilweise frei. Die Biegung des Siliziumsubstrats 26 ist in FIG 7 nicht dargestellt.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden, soweit alle Merkmale des unabhängigen Anspruchs enthalten sind .

## Patentansprüche

1. Verfahren zur Herstellung eines Mikrostrukturbauteils (4), wobei verfahrensgemäß
- in eine erste Oberfläche (23) eines scheibenartigen Siliziumsubstrats (26) eine Vielzahl von punktartigen Impfstrukturen (22) in einem entlang einer ersten und einer dazu senkrecht stehenden zweiten Substratrichtung (S1,S2) vorgegebenen Raster (28) eingebracht wird,
- die Impfstrukturen (22) in Tiefenrichtung (10) des Siliziumsubstrats (26) in einem ersten Ätzschritt (40) zu Bohrlöchern verlängert werden,
- eine der ersten Oberfläche (24) gegenüberliegende zweite Oberfläche (52) des Siliziumsubstrats (26) zur rückseitigen Öffnung der Bohrlöcher in einem zweiten Ätzschritt (50) zumindest teilweise entfernt wird,
- in einem dritten Ätzschritt (60) ein anisotrop wirksames Ätzmedium alternierend von beiden Oberflächen (24, 52) des Siliziumsubstrats (26) durch die Bohrlöcher gespült wird, so dass sich die in der ersten Substratrichtung (S1) nebeneinander angeordneten Bohrlöcher zu einem in der ersten Substratrichtung (S1) verlaufenden Spalt (62) verbinden,
wobei die Impfstrukturen (22) in der zweiten Substratrichtung (S2) mit einem ersten Abstand (A1) zueinander angeordnet werden, wobei die Impfstrukturen (22) in der ersten Substratrichtung (S1) zu Gruppen (32) zusammengefasst werden, die jeweils eine Unteranzahl der Impfstrukturen (22) umfassen, und wobei die Impfstrukturen (2) innerhalb der Gruppen (32) mit einem um einen Faktor von 0,2 bis 0,6, vorzugsweise von 0,3 bis 0,5, gegenüber dem ersten Abstand (A1) verringerten zweiten Abstand (A2) entlang der ersten Substratrichtung zueinander angeordnet werden, **dadurch gekennzeichnet, dass** die Gruppen (32) in der ersten Substratrichtung (S1) mit einem gegenüber dem zweiten Abstand (A1) vergrößerten dritten Abstand (A3) aufeinander folgend angeordnet werden.

2. Verfahren nach Anspruch 1,
wobei die Gruppen (32), die in der zweiten Substratrichtung (S2) aufeinander folgen, bezüglich ihrer Längserstreckung (L) entlang der ersten Substratrichtung (S1) zueinander versetzt angeordnet werden.

3. Verfahren nach Anspruch 2,
wobei die Gruppen (32) um etwa die Hälfte ihrer Längserstreckung (L) zueinander versetzt angeordnet werden.

4. Verfahren nach einem der Ansprüche 1 bis 3,
wobei für die Impfstrukturen (22) eine quadratische Grundfläche mit einer Kantenlänge kleiner oder gleich 2,5 µm herangezogen wird, und/oder wobei der erste Abstand (A1) 2 bis 20 µm beträgt, und/oder wobei die Längserstreckung (L) der Gruppen (32) zwischen 40 und 1300 µm, insbesondere zwischen 40 und 500 µm beträgt.

5. Verfahren nach einem der Ansprüche 1 bis 4,
wobei die Bohrlöcher bis zu einer Tiefe von etwa 500 µm ausgeformt werden.

6. Verfahren nach einem der Ansprüche 1 bis 5,
wobei als das in dem dritten Ätzschritt (60) eingesetzte anisotrope Ätzmedium eine Kaliumhydroxid enthaltende Lösung gewählt wird.

7. Verfahren nach einem der Ansprüche 1 bis 6,
wobei die Impfstrukturen (22) durch Ätzen mittels einer auf die erste Oberfläche (24) aufgebrachten Maskier-Schicht, die kreisförmige oder quadratische Löcher aufweist, ausgebildet werden.

8. Verfahren nach einem der Ansprüche 1 bis 7,
wobei das Mikrostrukturbauteil (4) nach dem dritten Ätzschritt (60) um eine entlang der ersten Substratrichtung (S1) liegenden Achse gebogen wird.

9. Verfahren nach einem der Ansprüche 1 bis 8,
wobei die Spalte (62) mit einem Röntgenstrahlen absorbierenden Material gefüllt werden, und/oder wobei in einem weiteren Ätzschritt ein in der zweiten Substratrichtung (S2) gesehen zentraler Bereich einer Rückseite des Siliziumsubstrats (26) entfernt wird.

## Claims

1. Method for producing a microstructure component (4), wherein, in accordance with the method
- a plurality of punctiform injection structures (22) are inserted in a predetermined grid (28) in a first substrate direction and a second substrate direction (S1, S2) standing at right angles thereto into a first surface (23) of a wafer-like silicon substrate (26),
- the injection structures (22) are lengthened into drilled holes in the depth direction (10) of the silicon substrate (26) in a first etching step (40),
- a second surface (52) of the silicon substrate (26) lying opposite the first surface (24) is at least partly removed for rear-side opening of the drilled holes in a second etching step (50),
- in a third etching step (60) an etching medium effective anisotropically is poured alternately through the drilled holes from both surfaces (24, 52) of the silicon substrate (26), so that drilled holes arranged next to one another in the first substrate direction (S1) connect to form a column (62) running in the first substrate direction (S1),
wherein the injection structures (22) in the second substrate direction (S2) are arranged at a first distance (A1) in relation to one another, wherein the injection structures (22) are combined into groups (32) in the first substrate direction (S1),which each comprise a subordinate number of the injection structures (22), and wherein the injection structures (2) within the groups (32) are arranged in relation to one another at a second distance (A2) reduced by a factor of 0.2-0.6, preferably of 0.3-0.5, compared to the first distance (A1) in the first substrate direction,
**characterised in that**
the groups (32) in the first substrate direction (S1) are arranged following one another at a third distance (A3) enlarged in relation to the second distance (A1).

2. Method according to claim 1,
wherein the groups (32), which follow each other in the second substrate direction (S2), are arranged in relation to their longitudinal extent (L) offset to one another in the first substrate direction (S1).

3. Method according to claim 2,
wherein the groups (32) are arranged offset in relation to one another by half of their longitudinal extent (L).

4. Method according to one of claims 1 to 3,
wherein a square basic surface with an edge length of less than or equal to 2.5 µm is employed for the injection structures (22), and/or wherein the first distance (A1) amounts to 2 to 20 µm, and/or wherein the longitudinal extent (L) of the groups (32) amounts to between 40 and 1300 µm, in particular to between 40 and 500 µm.

5. Method according to one of claims 1 to 4,
wherein the drilled holes are formed out to a depth of around 500 µm.

6. Method according to one of claims 1 to 5,
wherein a solution containing potassium hydroxide is selected as the anisotropic etching medium used in the third etching step (60).

7. Method according to one of claims 1 to 6,
wherein the injection structures (22) are embodied by means of etching a masking layer applied to the first surface (24), said masking layer having circular or square holes.

8. Method according to one of claims 1 to 7,
wherein the microstructure component (4) is bent about an axis lying along the first substrate direction (S1) after the third etching step (60).

9. Method according to one of claims 1 to 8,
wherein the columns (62) are filled with a material that absorbs x-rays, and/or wherein, in a further etching step, a central area of a rear side of the silicon substrate (26), seen in the second substrate direction (S2), is removed.

## Revendications

1. Procédé de fabrication d'un composant (4) microstructuré, dans lequel suivant le procédé
- on introduit dans une première surface (23) d'un substrat (26) en silicium de type à tranche, une pluralité de structures (22) ponctuelles d'inoculation en une trame (28) suivant une première direction (S1) de substrat et une deuxième direction (S2) du substrat, qui est perpendiculaire à la première direction (S1),
- on prolonge en des trous les structures (22) d'inoculation dans la direction (10) de la profondeur du substrat (26) en silicium dans un premier stade (40) d'attaque,
- on élimine au moins en partie dans un deuxième stade (50) d'attaque une deuxième surface (52), opposée à la première surface (24), du substrat (26) en silicium pour ouvrir les trous du côté arrière,
- dans un troisième stade (60) d'attaque, on fait couler dans les trous un milieu d'attaque efficace de manière anisotrope en alternance depuis les deux surfaces (24, 52) du substrat (26) en silicium de manière à relier les trous disposés les uns à côtés des autres dans la première direction (S1) du substrat en une fente (62) s'étendant dans la première direction (S1) du substrat,
dans lequel on dispose les structures (22) d'inoculation dans la deuxième direction (S2) du substrat à une première distance (A1) les unes des autres, dans lequel on rassemble les structures (22) d'inoculation dans la première direction (S1) du substrat en des groupes (32), qui comprennent respectivement un sous-nombre des structures (22) d'inoculation et dans lequel on met des structures (2) d'inoculation au sein des groupes (32) à une deuxième distance (A2) le long de la première direction du substrat les uns des autres diminuée d'un facteur de 0,2 à 0,6, de préférence de 0,3 à 0,5, par rapport à la première distance (A1),
**caractérisé**
**en ce que** l'on met successivement les groupes (32) dans la première direction (S1) du substrat à une troisième distance (A3) agrandie par rapport à la deuxième distance (A1).

2. Procédé suivant la revendication 1,
dans lequel on dispose de manière décalée les uns par rapport aux autres, le long de la première direction (S1) du substrat en ce qui concerne leur étendue (L) longitudinale les groupes (32) qui se suivent les uns les autres dans la deuxième direction (S2) du substrat.

3. Procédé suivant la revendication 2,
dans lequel on dispose les groupes (32) de manière décalée les uns par rapport aux autres d'environ la moitié de leur étendue (L) longitudinale.

4. Procédé suivant l'une des revendications 1 à 3,
dans lequel pour les structures (22) d'inoculation, on tire parti d'une surface de base carrée ayant une longueur de côté inférieure ou égale à 2,5 µm et/ou dans lequel la première distance (A1) va de 2 à 20 µm et/ou dans lequel l'étendue (L) longitudinale des groupes (32) est comprise entre 40 et 1300 µm, en étant notamment comprise entre 40 et 500 µm.

5. Procédé suivant l'une des revendications 1 à 4,
dans lequel on forme les trous jusqu'à une profondeur d'environ 500 µm.

6. Procédé suivant l'une des revendications 1 à 5,
dans lequel on choisit comme milieu d'attaque anisotrope utilisée dans le troisième stade (60) d'attaque une solution contenant de l'hydroxyde de potassium.

7. Procédé suivant l'une des revendications 1 à 6,
dans lequel on constitue les structures (22) d'inoculation par attaque au moyen d'une couche de masquage, qui est déposée sur la première surface (24) et qui a des trous circulaires ou carrés.

8. Procédé suivant l'une des revendications 1 à 7,
dans lequel on courbe le composant (4) microstructuré après le troisième stade (60) d'attaque autour d'un axe se trouvant dans la première direction (S1) du substrat.

9. Procédé suivant l'une des revendications 1 à 8,
dans lequel on remplit les fentes (62) d'un matériau absorbant les rayons X et/ou dans lequel, dans un autre stade d'attaque, on élimine une partie centrale, considérée dans la deuxième direction (S2) du substrat, d'une face arrière du substrat (26) en silicium.
